# EUROPÄISCHE PATENTSCHRIFT

(11) **EP 3 205 186 B1**
(45) Veröffentlichungstag und Bekanntmachung des Hinweises auf die Patenterteilung: **08.12.2021**
(21) Anmeldenummer: 15788342.2
(22) Anmeldetag: 09.10.2015
(51) Int. Cl.: H05H 1/24

(54) **VORRICHTUNG ZUM ERZEUGEN EINES KALTEN ATMOSPHÄRENDRUCKPLASMAS**
DEVICE FOR GENERATING A COLD ATMOSPHERIC PRESSURE PLASMA
DISPOSITIF DE PRODUCTION D'UN PLASMA FROID À PRESSION ATMOSPHÉRIQUE

(30) Priorität: 09.10.2014 DE 102014220488
(43) Veröffentlichungstag der Anmeldung: 16.08.2017
(73) Patentinhaber: Leibniz-Institut für Plasmaforschung und Technologie e.V., 17489 Greifswald (DE)
(72) Erfinder: MAHRENHOLZ, Carsten, 13359 Berlin (DE); GÜRA, Tobias, 17367 Eggesin (DE); BUSSIAHN, René, 14793 Greifswald (DE); KRAFCZYK, Stephan, 17489 Greifswald (DE); STIEBER, Manfred, 17489 Greifswald (DE); HORN, Stefan, 17438 Wolgast (DE); BRANDENBURG, Ronny, 17495 Groß Kiesow (DE); WELTMANN, Klaus-Dieter, 18609 Binz (DE); VON WOEDTKE, Thomas, 18519 Gerdeswalde (DE)
(74) Vertreter: Schulz Junghans Patentanwälte PartGmbB
(86) Internationale Anmeldenummer: PCT/EP2015/073484
(87) Internationale Veröffentlichungsnummer: WO 2016/055654

(56) Entgegenhaltungen:
- EP-A1- 2 170 022
- WO-A1-2014/040630
- WO-A2-2007/067924
- DE-A1- 10 136 403
- DE-A1-102008 030 913
- US-A1- 2012 271 225
- US-A1- 2014 182 879
- Katrin Oehmingen: "Plasma-Flüssigkeits-Wechselwirkungen", , 1 September 2013 (2013-09-01), XP055669939, Retrieved from the Internet: URL:https://epub.ub.uni-greifswald.de/fron tdoor/deliver/index/docId/1244/file/Disser tation_Plasma_Fluessigkeits_Wechselwirkung en.pdf [retrieved on 2020-02-19]
- Institute Leibniz: "Biennal Report 2012/2013", , 1 January 2014 (2014-01-01), XP055670043, Retrieved from the Internet: URL:https://www.inp-greifswald.de/fileadmi n/user_upload/documents/jahresbericht-2012 -2013_web.pdf [retrieved on 2020-02-19]

## Beschreibung

Die Erfindung betrifft eine Vorrichtung zum Erzeugen eines kalten Atmosphärendruckplasmas für die Behandlung von menschlichen und/oder tierischen Oberflächen nach Anspruch 1 sowie ein System nach Anspruch 10. In der Plasmamedizin sind in den letzten Jahren, aus der Zusammenarbeit von klassischer Plasmaphysik und den Lebenswissenschaften, erfolgversprechende Anwendungen bei der Behandlung von lebendem Gewebe entwickelt worden. Im Mittelpunkt der Plasmaanwendungen stand der Einsatz von nicht-thermischen Atmosphärendruckplasmen zur Dekontamination bis hin zur Sterilisation von lebendem Gewebe, also das Abtöten von Krankheitserregern an oder in einem lebenden Gewebe. Jedoch ist die Plasmabehandlung nicht auf die Desinfizierung und Sterilisation beschränkt. Weitere Anwendungen, die die besonderen Eigenschaften des Plasmas ausnutzen, können ebenfalls von vorteilhaften Wirkungen für die Medizin sein.

Ein möglicher Einsatz von Plasma ist die Förderung der Heilung von Wunden, wie z. B. chronischer und/oder postoperativer Wunden, aber auch die Behandlung von Verbrennungen, Abschürfungen, Augen- und Schleimhautinfektionen usw. Darüber hinaus ist auch ein Einsatz zur Desinfektion, Faltenbehandlung und/oder anderer kosmetischer Behandlungen denkbar. Insbesondere chronische Wunden, z. B. Diabetes-induzierte Wunden, erzeugen bei den betroffenen Patienten einen großen Leidensdruck und sind vielmals mit hohen Belastungen für den Patienten verbunden. Konventionelle Therapieansätze führen in vielen Fällen nicht zur gewünschten Heilung der Wunden, so dass oft nur der Status quo aufrechterhalten wird. Ein viel versprechender Ansatz zur Therapie von chronischen Wunden ist der Einsatz von kalten Plasmen, so genannten Atmosphärendruckplasmen.

DE 10 2008 030 913 A1 offenbart einen Wundschnellverband, der eine effiziente Entkeimung mittels Plasmas ermöglicht.

US 2012/271225 A1 offenbart eine Vorrichtung zur Behandlung von Bereichen menschlicher oder tierischer Hautoberflächen mittels eines kalten Atmosphärendruckplasmas.

Plasma gilt als vierter Zustand der Materie und besteht aus ionisiertem Gas mit physikalischen Besonderheiten. Plasma ist elektrisch geladenes Gas und leitet elektrischen Strom. Darüber hinaus enthält es eine Vielzahl von Radikalen, wie z. B. freie Elektronen, Ionen und/oder andere angeregte Spezies. Des Weiteren strahlt Plasma UV- und sichtbares Licht ab als auch weitere elektromagnetische Felder.

Durch die Entwicklung körperverträglicher Plasmen mit Temperaturen von weniger als 40°C ist ein neues, hochaktuelles Forschungsfeld entstanden - die Plasmamedizin. Diese "kalten Plasmen" sind die Grundlage vieler verschiedener Anwendungen in der Plasmamedizin. Bekannte, verfügbare Plasmaquellen haben im Rahmen klinischer Studien ihre Leistungsfähigkeit im Zusammenhang mit der Therapie verschiedener Hauterkrankungen und/oder der Behandlung von chronischen Wunden bewiesen. Ein wesentlicher Nachteil der bekannten Plasmaquellen besteht jedoch darin, dass bisher nur kleine Wundareale behandelt werden können, da die bekannten Plasmaquellen relativ klein sind. Darüber hinaus sind bekannte Plasmaquellen schwierig zu steuern, d. h. schwierig in der Dosierung und Handhabung.

Es besteht daher ein Bedarf an einer verbesserten, großflächigen Plasmaquelle für Atmosphärendruckplasmen, insbesondere für die Behandlung von menschlichen und/oder tierischen Oberflächen.

Aufgabe der vorliegenden Erfindung ist es, eine Vorrichtung zum Erzeugen eines kalten Atmosphärendruckplasmas für die Behandlung von menschlichen und/oder tierischen Oberflächen bereitzustellen, wobei eine großflächige, insbesondere ca. 400 cm² große, Plasmaquelle bereitgestellt werden soll. Darüberhinaus soll die Plasmaquelle sich flexibel an die Topographie der zu behandelnden Oberflächen anpassen, insbesondere an die unterschiedlichen Größen und Formen des Einsatzgebietes und der Anwendung. Auch ist es Aufgabe der Erfindung, ein System zum Betreiben einer (Plasma-)Vorrichtung zum Erzeugen eines großflächigen, kalten Atmosphärendruckplasmas für die Behandlung von menschlichen und/oder tierischen Oberflächen bereitzustellen.

Die vorstehende Aufgabe wird erfindungsgemäß durch eine Vorrichtung zum Erzeugen eines kalten Atmosphärendruckplasmas für die Behandlung von menschlichen und/oder tierischen Oberflächen nach Anspruch 1 sowie ein System nach Anspruch 10 gelöst. Gegenstände nach den abhängigen Unteransprüchen beschreiben bevorzugte Ausführungsbeispiele der Erfindung.

Ein erster Aspekt der Erfindung betrifft eine Vorrichtung, insbesondere eine Plasmavorrichtung, zum Erzeugen eines kalten Atmosphärendruckplasmas für die Behandlung von menschlichen und/oder tierischen Oberflächen, umfassend ein flexibles, flächiges Mehrschichtsystem mit einer der zu behandelnden Oberfläche zugewandten Seite und einer der zu behandelnden Oberfläche abgewandten Seite, wobei das Mehrschichtsystem die folgenden Schichten umfasst, nämlich eine erste Elektrodenschicht auf der abgewandten Seite des Mehrschichtsystems, eine zweite Elektrodenschicht auf der zugewandten Seite des Mehrschichtsystems, wobei die zweite Elektrodenschicht eine Vielzahl von Aussparungen aufweist oder gitterartig oder mäanderförmig ausgebildet ist, eine Dielektrikumsschicht, die zwischen der ersten Elektrodenschicht und der zweiten Elektrodenschicht angeordnet ist, eine erste Isolierschicht, die benachbart zur ersten Elektrodenschicht auf der von zu behandelnden Oberfläche abgewandten Seite des Mehrschichtsystems angeordnet ist, eine zweite Isolierschicht, die benachbart zur zweiten Elektrodenschicht auf der von zu behandelnden Oberfläche zugewandten Seite des Mehrschichtsystems angeordnet ist, und eine Abstandshalterschicht, die oberhalb auf der zweiten Isolierschicht auf der zugewandten Seite des Mehrschichtsystems angeordnet ist, und ferner konfiguriert ist, ein ausreichendes Gasvolumen zum Zünden des Plasmas bereitzustellen.

Im Folgenden wird das Konzept der Erfindung beispielhaft - ohne dabei einschränkend zu sein - beschrieben. Die erfindungsgemäße Vorrichtung, insbesondere Plasmavorrichtung, dient im Wesentlichen zur Behandlung von menschlichen und/oder tierischen Oberflächen, insbesondere der Behandlung von Wunden, wie z. B. chronischer und/oder postoperativer Wunden. Darüber hinaus dient sie aber auch zur Behandlung von Verbrennungen, Abschürfungen, Augen- und Schleimhautinfektionen usw. Auch der Einsatz zur Desinfektion, Faltenbehandlung und/oder anderer kosmetischer Behandlungen ist denkbar. Die Vorrichtung bedient sich einer speziellen, flexiblen (ggf. elastischen) Elektrodenanordnung mit mindestens zwei Elektrodenschichten, nämlich einer Hochspannungselektrode und einer Massenelektrode, zur Erzeugung eines flächigen Plasmas, insbesondere eines kalten Atmosphärendruckplasmas, mit Hilfe einer dielektrischen Schicht zwischen den beiden Elektroden. Die erfindungsgemäße Vorrichtung ist dabei konfiguriert, sich flexibel, insbesondere formschlüssig, an beliebig gekrümmte Oberflächen, z. B. im Gesicht eines Patienten, anzupassen und somit auch - für bekannte und unflexible Plasmaquellen - unzugängliche Hautareale, wie z. B. die Finger oder Zehen, für eine Plasmabehandlung zugänglich zu machen. Die Vorrichtung erzeugt ein flächiges Plasma auf einer Seite der Vorrichtung und wird dann mit dieser Seite auf die zu behandelnde Oberfläche, insbesondere auf die Wunde, gelegt, so dass die vorteilhaften Effekte/Eigenschaften des Plasmas auf die Oberfläche wirken bzw. mit ihr wechselwirken können.

Für das Bereitstellen einer flexiblen, großflächigen, dielektrisch behinderten Oberflächenentladung sind mindestens vier Schichten vorgesehen: zwei oder drei flexible Elektroden, nämlich eine erste und eine zweite Elektrodenschicht in einer jeweiligen Elektrodenebene, z. B Kupferfolien oder andere leitfähige Materialien, eine flexible Schicht mit einem flexiblen und/oder nicht flexiblen Funktionsdielektrikum zwischen den jeweiligen Elektroden, z. B. Silikon, Kapton, PVDF, ETFE und eine Abstandshalterschicht. Bevorzugt ist das Funktionsdielektrikum flexibel ausgebildet. Es können aber auch nicht flexible, dann jedoch flexibel miteinander verbundene, Materialien verwendet werden. Bevorzugt, aber ohne Einschränkung, wird ein Polymer verwendet. In anderen Ausführungsbeispielen werden Elastomere, Textilgewebe oder z. B. Keramiken, die in einer Silikonmatrix eingebettet sind oder offenporige Schäume, wie z. B. Chitinstoffe, wie Chitosan oder Chitosan-Pflaster, verwendet.

Zum Zünden des Plasmas ist an einer der beiden Elektroden eine Hochspannung angelegt, wobei die zweite Elektrode dann auf Erd- oder Massenpotential liegt und damit eine Gegenelektrode für die Hochspannungselektrode bildet. Zwischen den beiden Elektroden liegt dann ein Hochspannungsfeld, wobei ein Kurzschluss in Form eines Lichtbogens zwischen den Elektroden durch die Dielektrikumsschicht verhindert oder unterbunden wird. Stattdessen bildet sich ein großflächiges, dielektrisch behindertes Atmosphärendruckplasma aus. Da die Plasmaeigenschaften stark von der Gasraumdicke, insbesondere von dem Gasvolumen zwischen der Masseelektrode und der zu behandelnden Oberfläche, insbesondere der Haut, abhängen, ist eine Abstandshalterschicht vorgesehen, die ein zuverlässiges und reproduzierbares Bereitstellen einer ausreichenden Gasmenge für das Erzeugen eines Plasmas mit definierten Plasmaeigenschaften erlaubt. Das zu ionisierende Gas ist dabei entweder ein zugeführtes Arbeitsgas oder Gasgemisch und/oder die Umgebungs- oder Außenluft. Die Abstandshalterschicht kann - ohne Einschränkung der Erfindung - hierbei in vielfältiger Art und Weise ausgestaltet sein, beispielweise mit Stegen, Aussparungen, Noppen, Schäume von herkömmlichen Wundauflagen und/oder herkömmliche Wundauflage usw., die dann jeweils unterschiedliche Formen und Dicken haben können. Beispielsweise kann die Abstandshalterschicht auch in Form eines selbstklebenden Randes ausgebildet sein, mit dem die Vorrichtung am Patienten befestigt wird.

Die Elektroden, sind bevorzugt mit leitfähigen Materialien, insbesondere mit Metallen, beispielsweise in Form von dünnen Metallschichten, -folien, -gittern und/oder mit leitfähigen Polymerschichten gebildet.

Diese und weitere bevorzugte Ausgestaltungsformen der Erfindung sind Gegenstand der Unteransprüche und geben im Einzelnen vorteilhafte Möglichkeiten an, wie die Erfindung im Rahmen der Aufgabenstellung sowie hinsichtlich weiterer Vorteile zu realisieren beziehungsweise auszugestalten ist.

Bevorzugt sieht eine Ausgestaltung vor, dass die Abstandshalterschicht mit zumindest einem Polymer, insbesondere einem Elastomer, und/oder einem Textilgewebe und mit Dicken von 0,5 mm bis 5 mm gebildet ist.

Das Mehrschichtsystem der vorliegenden Erfindung weist zusätzlich eine erste Isolierschicht auf, wobei die erste Isolierschicht benachbart zur ersten Elektrodenschicht auf der von der zu behandelnden Oberfläche abgewandten Seite des Mehrschichtsystems angeordnet ist. Die erste Isolierschicht ist auf der von der zu behandelnden Oberfläche abgewandten Seite des Mehrschichtsystems angeordnet und weist in einer bevorzugten Weiterbildung eine Dicke zwischen 0,5 mm und 5 mm, bevorzugt von 2 mm auf. Die erste Isolierschicht dient im Wesentlichen zur elektrischen Isolierung der ersten Elektrodenschicht, die bevorzugt als Hochspannungselektrodenschicht ausgebildet ist, d. h. als eine Elektrodenschicht an der eine Hochspannung anliegt. In einer weiteren Weiterbildung dieser Ausgestaltung ist die erste Elektrodenschicht an mehreren Seiten, insbesondere allseitig, isoliert.

Das Mehrschichtsystem der vorliegenden Erfindung weist zusätzlich eine zweite Isolierschicht auf, wobei die zweite Isolierschicht benachbart zu der zweiten Elektrodenschicht auf der von der zu behandelnden Oberfläche zugewandten Seite des Mehrschichtsystems angeordnet ist. Bevorzugt ist hierbei vorgesehen, dass die zweite Isolierschicht eine Dicke zwischen 10 µm bis 300 µm aufweist.

Eine Weiterbildung kann vorsehen, dass das Mehrschichtsystem zusätzlich eine dritte Isolierschicht aufweist, wobei die dritte Isolierschicht benachbart zu der Abstandshalterschicht auf der von der zu behandelnden Oberfläche zugewandten Seite des Mehrschichtsystems angeordnet ist. Bevorzugt ist die Isolierschicht mit einem haut- und/oder wundverträglichen Material, bevorzugt mit antiseptischen und/oder atraumatischen Eigenschaften, gebildet. In einer weiteren vorteilhaften Ausgestaltung weist die dritte Isolierschicht eine Dicke zwischen 50 µm bis 300 µm, bevorzugt von 200 µm auf.

In einer Ausgestaltung hat das Mehrschichtsystem Abmaße, die eine Länge und eine Breite zwischen jeweils 5cm bis 25cm aufweisen.

Eine besonders vorteilhafte Ausgestaltung betrifft ein Mehrschichtsystem, wobei die erste Elektrodenschicht durchgängig oder mit einer Vielzahl von Aussparungen gebildet ist.

Bei einer zweckmäßigen Ausgestaltung kann es vorgesehen sein, dass die Aussparungen in der ersten und/oder in der zweiten Elektrodenschicht zwischen den leitfähigen Strukturen loch-, streifen-, mäander, waben-, kreisförmig und/oder quadratisch ausgebildet sind. Beispielsweise können die kreisförmige und/oder wabenförmige Aussparungen als Löcher mit einem Durchmesser von 3 mm bis 5 mm gebildet sein, die dann reihenförmig und/oder versetzt nebeneinander angeordnet sind. In einem anderen Ausführungsbeispiel sind quadratische Aussparungen mit Abmaßen von 3 mm x 3 mm bis 5 mm x 5 mm, bevorzugt von 4 mm x 4 mm vorgesehen, wobei die Stege zwischen den Aussparungen eine Breite zwischen 0,1 mm und 5 mm aufweisen können. Wiederum in einer anderen Ausführungsform kommen streifenförmige Aussparungen mit einer Breite zwischen 1 mm und 10 mm, bevorzugt mit einer Breite von 6 mm, zum Einsatz. Die streifenförmigen Aussparungen sind dann beispielsweise parallel, kreisförmig, halbkreisförmig, spiralförmig und/oder mäanderförmig angeordnet.

Eine bevorzugte Weiterbildung sieht vor, dass die Vorrichtung einen Informationsträger, beispielsweise einem Chip oder ein Etikett oder ein Label oder ein sonstiges Informations- und Speichermedium umfasst, auf dem Betriebsparameter zum Betreiben der Vorrichtung gespeichert sind. Insbesondere bei einer mehrfachen Verwendung der Vorrichtung ist es vorteilhaft, dass die Vorrichtungsspezifischen Daten, insbesondere die Betriebsparameter zum Betreiben der Vorrichtung, in einem Informations- und Speichermedium, beispielsweise einem Mikrochip, auf oder an der Vorrichtung abgelegt bzw. gespeichert sind, so dass diese vor und/oder während des Betriebes des Vorrichtung ausgelesen werden können. Mögliche Daten, die bevorzugt gespeichert sind, können Daten über ein Behandlungsschema, die Benutzungsdauer, Lebenszeit, Pulsmuster, Intensität (Amplitude der Versorgungsspannung), eine ID oder Seriennummer der Vorrichtung, die Anzahl der bisherigen Anwendungen, Hygienestatus (nicht-steril, benutzt, desinfiziert, steril usw.), Fehler oder Fehlermeldungen während der Verwendung der Vorrichtung (z. B. Durchschläge oder Kurzschlüsse, Schwankungen der Betriebsparameter), Verwendbarkeit/Verwendungsstatus (z. B. gültig oder ungültig) sein.

Das Auslesen des Informationsträgers oder Speichermediums kann beispielsweise kabelgebunden, optisch oder mittels Funktechnologie erfolgen. Darüberhinaus ist mit einem derartigen Informationsträger auch ein Sicherheitselement bereitgestellt, dass beispielsweise einen Betrieb der Vorrichtung nur dann freigibt, wenn die notwendigen Voraussetzungen gegeben sind. Auch können mit Hilfe des Informationsträgers mehrfach Verwendungen einer Vorrichtung unterbunden werden, beispielweise dann, wenn eine Vorrichtung aus hygienischen Gründen nur einmal verwendet werden darf. Für solche Einweg-Vorrichtung ist aus Kostengründen eine Barcode- bzw. QR-Code-Lösung zu bevorzugen. In diesem Fall wären beispielsweise die Behandlungsparameter (Betriebsparameter und eine zulässige Indikation) zu kodieren, so dass beispielsweise die Echtheit (Originalität) der Vorrichtung überprüft werden kann. Diese Funktionalität kann beispielsweise mittels eines verschlüsselten Nummernkreises realisiert sein.

Ein erster Aspekt der Offenbarung, der nicht durch die beanspruchte Erfindung abgedeckt ist, betrifft ein Kabel zum Verbinden mit einer Vorrichtung nach dem ersten Aspekt der Erfindung, wobei das Kabel einen Stecker aufweist, der konfiguriert ist, eine steckbare Hochspannungsverbindung zwischen der Vorrichtung und dem Kabel bereitzustellen.

Das Kabel dient einerseits dazu die (Plasma-)Vorrichtung nach dem ersten Aspekt der Erfindung mit Hochspannung zu versorgen, andererseits soll das Kabel bevorzugt auch dazu ausgebildet sein, steuertechnische Signale zwischen einer Versorgungseinheit und der Vorrichtung zu übertragen. Die Signale sollen dabei bi-direktional geleitet werden, beispielsweise von der Plasmavorrichtung zu einer Versorgungs-/Steuereinheit und umgekehrt.

Die wesentliche Aufgabe des Kabels ist es aber, die zur Erzeugung eines Plasmas notwendige Hochspannung von einem Hochspannungsgenerator zur Vorrichtung zu übertragen. Wesentliche Funktion des Kabels ist hierbei die sichere Übertragung der Hochspannung, die sichere Isolation nach Außen (Berührungsschutz) und nach Innen (Durchschlagfestigkeit). Darüberhinaus muss das Kabel flexibel ausgebildet sein. Mit dem Kabel wird also eine elektrische Hochspannungsverbindung zwischen Vorrichtung und Hochspannungsgenerator bereitgestellt, wobei das Kabel mindestens einen HV-Leiter, einen Isolator und eine Masseleitung umfasst. Bevorzugt ist aus Gründen der elektrischen Sicherheit und der EMV eine zusätzliche Schirmung vorgesehen, die entweder mit der Masseleitung identisch oder unabhängig mit dem Schutzleiter (PE) verbunden ist. Die Art der Schirmung hängt in erster Linie von den auftretenden Störungen ab. Besonders gute Schirmleistung lässt sich durch eine doppelte Schirmung (metallische bzw. metallisierte Folie und ein Schirmgeflecht) erzielen. Für die äußere Isolierung des Hochspannungskabels ist ein biokompatibles, desinfizierbares Material bevorzugt, da in der Praxis das Kabel häufig mittels eines Pflasters am Körper des Patienten fixiert wird. Darüberhinaus können weitere elektrische (Steuer-)Leitungen, beispielsweise eine Datenleitung zur Kommunikation mit einem in der Vorrichtung integrierten Speicherchip, vorgesehen sein. Ergänzend oder alternativ, können auch eine doppelte Schirmung und/oder Ferritkerne zur EMV Verbesserung, Gasleitung(en) zur Zuführungen von Arbeitsgasen, wie z. B. angefeuchteter Luft und/oder Edelgas(en) sowie spezieller Gasgemische oder zur Abführung (Absaugleitungen) unerwünschter Gaskomponenten wie z. B. Ozon vorgesehen sein. Zur Verbesserung der EMV-Eigenschaften kann es notwendig sein zwischen der Vorrichtung gemäß dem ersten Aspekt der Erfindung und dem HV-Kabel ein oder mehrere weitere elektronische Bauelemente, wie beispielsweise Spulen, Kondensatoren und Filter, zu integrieren. Desweiteren können zwischen der Vorrichtung und dem HV-Kabel auch obengenannte Maßnahmen zur Verbesserung der elektrischen Sicherheit und der EMV vorgesehen sein.

Der Anschluss des Kabels an der Vorrichtung kann wahlweise fest oder über ein Stecksystem erfolgen. Die Stecker-Variante erlaubt ein einfaches Austauschen des Kabels bei Defekten und/oder für Reinigungs-/Desinfektionszwecke. Des Weiteren sind alle möglichen Kabellängen von 1 m bis 20 m vorgesehen.

Bevorzugt sieht eine Ausgestaltung vor, dass das Kabel eine Klemmvorrichtung aufweist, wobei die Klemmvorrichtung zwischen einer offenen Position und einer geschlossenen Position verlagerbar ist und in der geschlossenen Position die Vorrichtung elektrisch mit dem Kabel verbunden und in der offenen Position die Vorrichtung von dem Kabel elektrisch getrennt ist. Bevorzugt sind Kabel und Klemmvorrichtung als (Hochspannungs)Einwegprodukt ausgebildet, wobei eine Entwertung des Einmalproduktes nach der Behandlung vorgesehen ist, beispielweise dann, wenn diese aus hygienischen Gründen nur einmal verwendet werden darf.

Bevorzugt sind bei dem Stecker des Kabels eine Masse- und Hochspannungskontaktierung seitlich versetzt nebeneinander angeordnet.

Ein zweiter Aspekt der Offenbarung, der nicht durch die beanspruchte Erfindung abgedeckt ist, betrifft eine Generatoreinheit zum Bereitstellen einer Hochspannung zum Erzeugen eines kalten Atmosphärendruckplasmas mit einer Vorrichtung nach dem ersten Aspekt der Erfindung für die Behandlung von menschlichen und/oder tierischen Oberflächen, wobei die Generatoreinheit konfiguriert ist, die Vorrichtung zu steuern.

Die Generatoreinheit stellt eine zentrale Steuereinheit für die (Plasma-)Vorrichtung dar und dient in erster Linie dazu, die Hochspannung mittels eines Hochspannungsgenerators für die Vorrichtung bereitzustellen. Die Generatoreinheit umfasst einen Hochspannungsgenerator mit Steuereinheit und zumindest einen Anschluss für das (Versorgungskabel der (Plasma-)Vorrichtung sowie einen Netzanschluss mit Netzschalter (Hauptschalter) und ggf. integriertem Netzfilter und einem Kühler zur Kühlung der Elektronik. Optional ist ein Gasanschluss vorgesehen mit einem Gas-Flow Controller und/oder einem Kompressor und/oder einer Absaugvorrichtung. Darüberhinaus sind bevorzugt weitere Steuereinheiten, Mikro-Controller, Platinen, Displays, insbesondere Touchscreen-Displays, Folientastaturen usw. zum Betreiben der Generatoreinheit vorgesehen.

Bei einer zweckmäßigen Ausgestaltung kann es vorgesehen sein, dass die Generatoreinheit zusätzlich konfiguriert ist, Betriebsparameter zum Steuern der Vorrichtung automatisch aus einem Informationsträger, insbesondere einen Chip, ein Etikett und/oder ein sonstiges Informations- und Speichermedium, in oder an der Vorrichtung auszulesen. In Abhängigkeit von der Art der angeschlossenen Vorrichtung, insbesondere abhängig von der Größe- und/oder den spezifischen Behandlungsparametern, werden dann die entsprechenden Betriebsparameter aus einem Informationsträger gelesen und der Generatoreinheit bereitgestellt. Diese können beispielsweise dann auch auf dem Display, insbesondere Touchscreen-Display, der Generatoreinheit angezeigt werden.

Ein zweiter Aspekt der Erfindung betrifft ein System mit einer Vorrichtung nach dem ersten Aspekt der Erfindung, einem Kabel nach dem ersten Aspekt der Offenbarung und einer Generatoreinheit nach dem zweiten Aspekt der Offenbarung.

Ausführungsbeispiele der Erfindung werden nachfolgend anhand der Figuren beschrieben. Diese sollen die Ausführungsbeispiele nicht notwendigerweise maßstäblich darstellen, vielmehr sind die Figuren in schematischer und/oder leicht verzerrter Form ausgeführt.

Hierbei sind identische und/oder ähnliche Merkmale mit identischer oder ähnlicher Funktion, dort wo sinnvoll, mit gleichen Bezugszeichen versehen. Weitere Vorteile, Merkmale und Einzelheiten der Erfindung ergeben sich aus der nachfolgenden Beschreibung der bevorzugten Ausführungsbeispiele sowie anhand der Figuren.

Der Schutzumfang der vorliegenden Erfindung wird jedoch durch die Patentansprüche bestimmt.

Im Einzelnen zeigen:
- Fig. 1:: eine perspektivische, schematische Darstellung einer Vorrichtung zur Erzeugung eines kalten Atmosphärendruckplasmas für die Behandlung von Oberflächen,
- Fig. 2:: eine Explosionsdarstellung der in der Fig. 1 dargestellten Vorrichtung,
- Fig. 3:: eine schematische Darstellung einer bevorzugten Ausführungsform für ein Kabel mit einem Stecker,
- Fig. 4:: eine schematische Darstellung einer Ausführungsform für ein Steckergehäuse,
- Fig. 5:: eine perspektivische, schematische Darstellung einer Vorrichtung zum Behandeln von Oberflächen und einem Stecker,
- Fig. 6:: eine bevorzugte Ausgestaltungsform für eine Klemmvorrichtung für einen Stecker,
- Fig. 7:: eine bevorzugte Ausgestaltungsform für einen Generator,
- Fig. 8:: eine schematische Darstellung für eine bevorzugte Ausführungsform für ein System mit einer Vorrichtung, ein Generator und eine Kabel zum Verbinden der Vorrichtung mit dem Generator, und
- Fig. 9:: bevorzugte Ausgestaltungsformen, insbesondere Aussparungen in der Elektrodenschicht, für eine Vorrichtung zum Behandeln von Oberflächen.

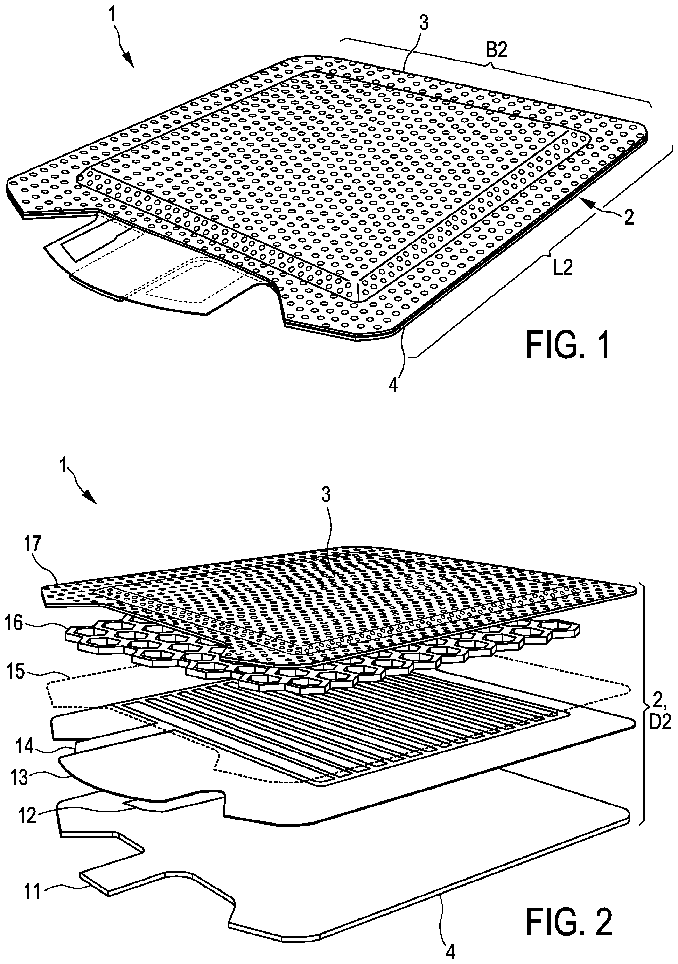

Fig. 1 zeigt eine perspektive Darstellung einer Vorrichtung 1 zur Erzeugung eines kalten Atmosphärendruckplasmas. Die dargestellte Vorrichtung 1, auch Plasmapatch genannt, ist eine großflächige Plasmaquelle für die Behandlung von menschlichen und/oder tierischen Oberflächen, insbesondere zur Behandlung von Wunden und Förderung der Wundheilung. Die Vorrichtung bedient sich einer speziellen, flexiblen Elektrodenanordnung mit zwei Elektrodenschichten, nämlich eine Hochspannungselektrode und einer Massenelektrode, zur Erzeugung eines flächigen Plasmas mittels einer dielektrischen Schicht zwischen den beiden Elektroden, wobei die Vorrichtung konfiguriert ist, sich flexibel auf beliebig gekrümmte Oberflächen auflegen zu lassen und somit für die Plasmabehandlung erkrankter/geschädigter Hautareale geeignet ist. Die Vorrichtung 1 erzeugt dabei ein flächiges Plasma auf einer Seite der Vorrichtung, wobei die Vorrichtung dann mit dieser Seite auf die zu behandelnden Oberfläche, insbesondere auf eine Wunde, gelegt wird, so dass die vorteilhaften Effekte/Eigenschaften des Plasmas auf die Oberfläche wirken können.

Die Vorrichtung 1 umfasst ein flexibles, flächiges Mehrschichtsystem 2 mit einer der zu behandelnden Oberfläche zugewandten Seite 3 und einer der zu behandelnden Oberfläche abgewandten Seite 4. Das Mehrschichtsystem 2 ist dabei mit mehreren Schichten gebildet, die im Einzelnen in der Fig. 2 im Detail beschrieben werden. Die äußeren Maße, insbesondere die Abmaße des Mehrschichtsystems 2, weisen eine Länge L2 und eine Breite B2 zwischen 5 cm bis 25 cm, bevorzugt von 20 cm x 20 cm auf. Ohne Einschränkung der Erfindung können aber auch andere Formen also nicht nur quadratische Formen vorgesehen sein. Bevorzugt passen diese sich dann formschlüssig an die Oberfläche z. B. das Gesicht eines Patienten an. Ebenfalls vorgesehen sind Vorrichtungen in Form von Manschetten, Unterlagen, Bettbezügen, Bettlaken oder dergleichen.

Fig. 2 zeigt eine Explosionsdarstellung der in der Fig. 1 dargestellten Vorrichtung 1, mit einem Mehrschichtsystem 2. Das Mehrschichtsystem 2 umfasst dabei die folgenden Schichten, nämlich (von unten):
- eine erste Isolierschicht 11,
- eine erste Elektrodenschicht 12,
- eine Dielektrikumsschicht 13,
- eine zweite Elektrodenschicht 14,
- eine zweite Isolierschicht 15,
- eine Abstandshalterschicht 16, und
- eine dritte Isolierschicht 17.

Die erste Isolierschicht 11 ist dabei auf der von der zu behandelnden Oberfläche abgewandten Seite 4 des Mehrschichtsystem 2 angeordnet und weist eine Dicke zwischen 0,5 mm und 4 mm, bevorzugt von 2 mm auf. Die erste Isolierschicht 11 dient im Wesentlichen zur Isolierung der ersten Elektrodenschicht 12, die bevorzugt als Hochspannungsschicht gebildet ist, d. h. eine Elektrodenschicht, an die eine Hochspannung angelegt wird.

Die Dielektrikumsschicht 13 ist zwischen der ersten Elektrodenschicht 12 und der zweiten Elektrodenschicht 14 angeordnet, wobei die zweite Elektrodenschicht 14 bevorzugt als eine Massenelektrodenschicht ausgebildet ist. Die Dielektrikumsschicht 13 verhindert dabei im Wesentlichen einen Kurzschluss zwischen der ersten und zweiten Elektrodenschicht, insbesondere in Form eines Lichtbogens.

Weiterhin ist in der vorliegenden Erfindung auf der zweiten Elektrodenschicht 14 eine zweite Isolierschicht 15 angeordnet, die in einer bevorzugten Ausgestaltungsform eine Dicke zwischen 10 µm und 300 µm aufweist.

Oberhalb der zweiten Elektrodenschicht 14 oder der zweiten Isolierschicht 15, also auf der von der zu behandelnden Oberfläche zugewandten Seite 3 des Mehrschichtsystems 2 ist dann die Abstandshalterschicht 16 angeordnet, die dafür Sorge trägt, dass ausreichend Gasvolumen bereitgestellt wird, so dass ein Plasma zünden kann.

Abschließend ist auf der zu behandelnden Oberfläche zugewandten Seite 3 des Mehrschichtsystems 2 und oberhalb der Abstandshalterschicht 16 eine dritte Isolierschicht 17 angeordnet, die eine Dicke zwischen 100 µm bis 300 µm, bevorzugt von 200 µm aufweist und die im direkten Kontakt mit der zu behandelnden Oberfläche steht. Bevorzugt ist die dritte Isolierschicht 17 dann mit einem haut- und/oder wundverträglichen Material bevorzugt mit antiseptischen und/oder atraumatischen Eigenschaften, gebildet.

Vorliegend, wie in der Fig. 2 dargestellt, ist die zweite Elektrodenschicht 14 mit einer Vielzahl von Aussparungen, insbesondere gitterartig, ausgebildet. Ohne Einschränkung der Erfindung können die Aussparungen aber auch loch-, streifen-, mäander-, waben-, kreisförmig und/oder quadratisch ausgebildet sein.

Weiterhin kann auch die Abstandshalterschicht 16 wabenförmig gebildet sein, wobei die Abstandshalterschicht 16 - ohne Einschränkung der Erfindung - auch durch Vorsprünge oder Stege realisiert sein kann. Mögliche Materialien für die Abstandshalterschicht 16 sind Polymere, Elastomere und/oder Silikone oder dergleichen. Grundsätzlich können eine Vielzahl von möglichen Materialien verwendet werden, wie zum Beispiel anorganische oder organische Materialien, insbesondere natürliche und/oder synthetische Materialien, wie Thermoplaste, Duroplaste und/oder Elastomere. Für weitere mögliche Materialien wird beispielhaft auch auf das Buch "Kunststoff-Taschenbuch" (28. Auflage) von Karl Oberbach und Hansjürgen Saechtling verwiesen. In einer bevorzugten Ausgestaltungsform ist die Abstandshalterschicht mit Vorsprüngen und/oder Stegen gebildet, die eine Höhe zwischen 0,5 mm und 10 mm aufweisen.

Insgesamt weist das in der Fig. 2 dargestellte Mehrschichtsystem eine Dicke d2 von 2 mm bis 15 mm auf. Hierbei ist es vorgesehen, dass die Schichten, die mit der zu behandelnden Oberfläche in direktem Kontakt sind aus einem hitzebeständigen, biokompatiblen und chemisch beständige Kunststoffe gebildet sind.

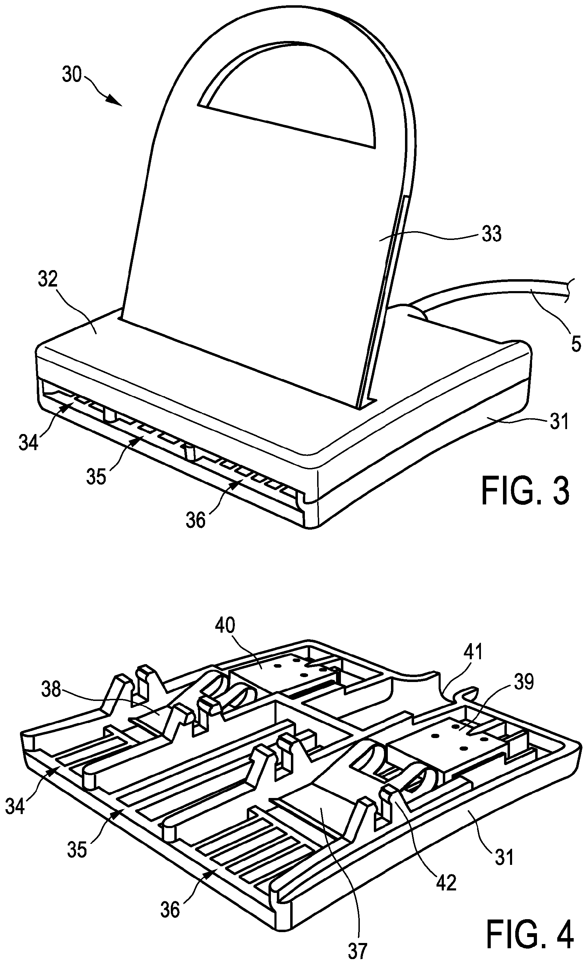

Fig. 3 zeigt eine schematische Darstellung einer bevorzugten Ausführungsform für ein Kabel 5 mit einem Stecker 30. Die wesentliche Aufgabe des Kabels 5 ist es, die zur Erzeugung eines Plasmas notwendige Hochspannung von einem Hochspannungsgenerator (nicht dargestellt) zur Vorrichtung zu übertragen, wobei das Kabel mindestens einen HV-Leiter, einen Isolator und eine Masseleitung (nicht dargestellt) umfasst. Der Anschluss des Kabels an der Vorrichtung kann wahlweise fest oder über ein Stecksystem erfolgen, wobei die Stecker-Variante ein einfaches Austauschen des Kabels bei Defekten und/oder für Reinigungs-/ Desinfektionszwecke erlaubt. Des Weiteren sind alle möglichen Kabellängen von 1 m bis 20 m vorgesehen.

Die in der Fig. 3 gezeigte Ausführungsform zeigt ein Kabel mit einem möglichen Stecker, wobei der Stecker 30 ein unteres Steckergehäuse 31, ein oberes Steckergehäuse 32 und eine Klemmvorrichtung 33 umfasst. Darüberhinaus umfasst der Stecker 30 einen ersten Anschluss 34 für die erste Elektrode einer Vorrichtung gemäß der vorliegenden Erfindung (nicht dargestellt), einen zweiten Anschluss 36 für eine zweite Elektrode der Vorrichtung und einen weiteren Anschluss 35 für Steuersignale und oder beispielsweise zum Auslesen von Betriebsparametern für die Vorrichtung, welche beispielsweise auf einem Chip in der Vorrichtung gespeichert sind.

Die dargestellte Klemmvorrichtung 33 des Steckers 30 ist zwischen einer ersten offenen Position und einer zweiten geschlossenen Position verlagerbar. Hierbei ist die Vorrichtung (nicht dargestellt) in der geschlossenen Position elektrisch mit dem Kabel 5 verbunden und in der offenen Position ist die Vorrichtung dann elektrisch vom Kabel 5 getrennt.

Fig. 4 zeigt eine mögliche Ausgestaltungsform des Inneren eines Steckers 30, wie er beispielsweise in der Fig. 3 dargestellt und beschrieben ist. Der Stecker, insbesondere das untere Steckergehäuse 31, umfasst eine erste Klemmzunge 37 und eine zweite Klemmzunge 38, die jeweils konfiguriert sind, die erste oder zweite Elektrode der Vorrichtung (nicht dargestellt) mit einem Hochspannungsanschluss 39 oder einem Massenanschluss 40 des Kabels (nicht dargestellt) zu verbinden, wobei das Kabel über den Kabelanschluss 41 mit dem Stecker 30 verbunden wird. Weiterhin umfasst der Stecker 30 mindestens ein Gelenk 42. Mittels des Gelenkes 42 kann die Klemmvorrichtung 33 von der offenen in die geschlossene Position verlagert werde und umgekehrt. Hierbei wirkt die Klemmvorrichtung mit der ersten Klemmzunge 37 und der zweiten Klemmzunge derart zusammen, dass in der geschlossenen Position die erste und/oder zweite Elektrode der Vorrichtung mit dem Hochspannungsanschluss 39 oder dem Massenanschluss 40 des Kabels elektrisch verbunden werden. In der offenen Position der Klemmvorrichtung geben die Klemmzungen dann die jeweiligen Elektroden frei, so dass diese nicht mehr elektrisch mit dem Kabel verbunden sind. Stecker und Klemmvorrichtungen sind dabei derart ausgebildet, dass sie Hochspannungsanforderungen genügen.

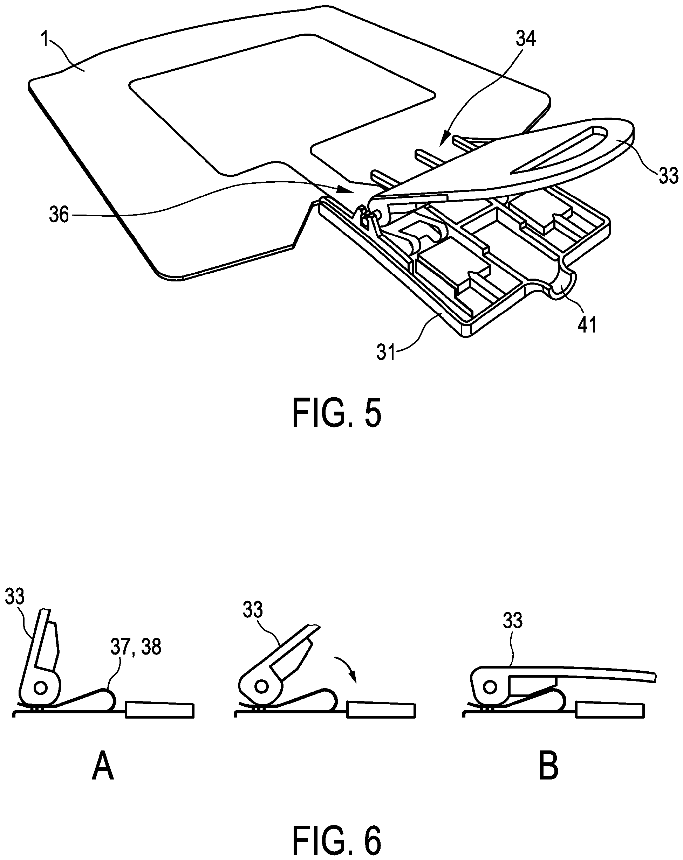

Fig. 5 zeigt eine perspektivische, schematische Darstellung einer Vorrichtung 1 zum Behandeln von Oberflächen, wie sie beispielsweise in der Fig. 1 dargestellt und beschrieben ist, zusammen mit einem Stecker 30, wie er beispielsweise in den Fig. 3 und 4 dargestellt und beschrieben ist.

Fig. 6 zeigt eine bevorzugte Ausgestaltungsform für eine Klemmvorrichtung 33 für einen Stecker (nicht dargestellt), wie er in den Fig. 3 und 4 beschrieben ist. Schematisch dargestellt ist die Verlagerungsbewegung der Klemmvorrichtung 33 von der offenen Position A in die geschlossene Position B, wobei der Pfeil die Bewegungsrichtung der Klemmvorrichtung während der Verlagerung anzeigt.

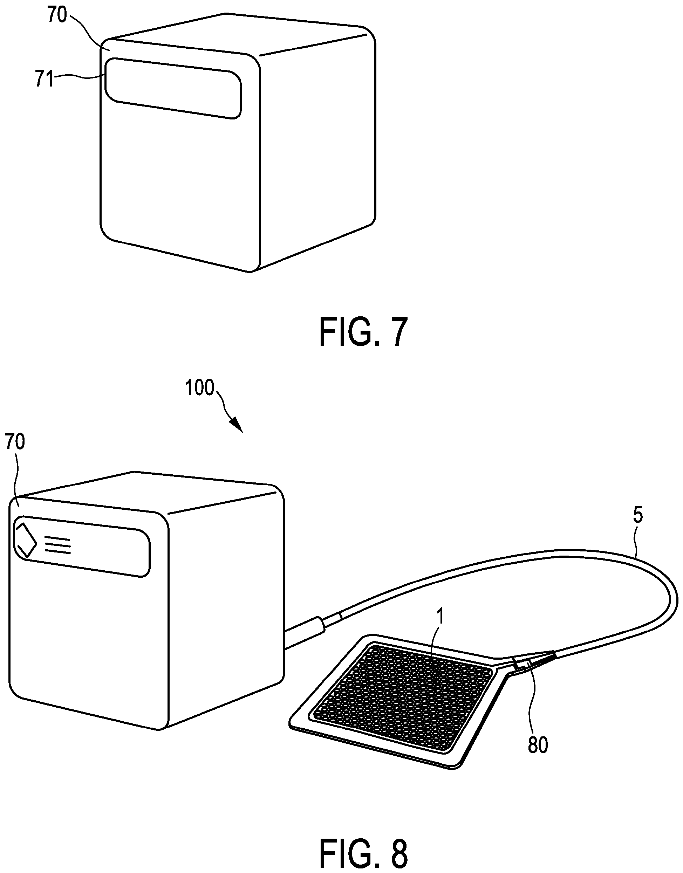

Fig. 7 zeigt eine bevorzugte Ausgestaltungsform für eine Generatoreinheit 70 zum Bereitstellen einer Hochspannung zum Betreiben einer Vorrichtung, wie sie beispielsweise in den Fig. 1 und 2 dargestellt und beschrieben ist. Die Generatoreinheit 70 dient in erster Linie dazu, die Hochspannung mittels eines Hochspannungsgenerators für die Vorrichtung bereit zustellen. Die Generatoreinheit 70 umfasst hierfür einen Hochspannungsgenerator mit Steuereinheit und zumindest einen Anschluss für das (Versorgungs-)Kabel der (Plasma-)Vorrichtung sowie einen Netzanschluss mit Netzschalter (nicht dargestellt).

Optional ist ein Gasanschluss mit einem Gas-Flow Controller und/oder einem Kompressor und/oder einem Filter und/oder einer Absaugvorrichtung vorgesehen. Darüberhinaus sind bevorzugt ein Display 71, weitere Steuereinheiten, Micro-Controller, Platinen usw. zum Betreiben der Generatoreinheit vorgesehen.

Auch ist die Generatoreinheit 70 konfiguriert, mit einer Vorrichtung gemäß der Erfindung zusammenzuwirken bzw. zu kommunizieren, insbesondere hierbei automatisch die Betriebsparameter einer bestimmten Vorrichtung auszulesen, welche beispielsweise auf einem Chip 80 (vgl. auch Fig. 8) in der Vorrichtung gespeichert sind. Basierend auf den ausgelesenen Betriebsparametern kann dann die Generatoreinheit automatisch eingestellt werden, ohne dass die Parametereinstelllung von einem Benutzer an der Generatoreinheit manuell eingestellt werden muss. Auch können die Betriebsparameter dann auf dem Display oder Bildschirm 71 der Generatoreinheit 70 angezeigt werden.

Fig. 8 zeigt eine schematische Darstellung für eine bevorzugte Ausgestaltungsform für ein System 100 mit einer Vorrichtung, wie sie beispielsweise in den Figuren 1 und 2 gezeigt ist, einer Generatoreinheit, wie sie in der Fig. 7 beschrieben ist, wobei Vorrichtung und Generatoreinheit mittels eines Kabels 5 steuerbar verbunden sind.

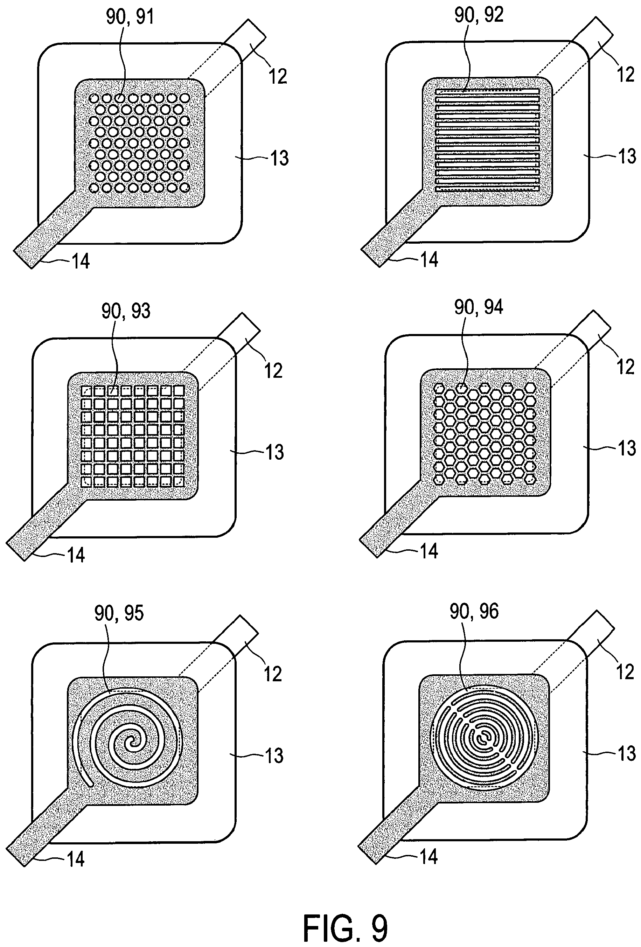

Fig. 9 zeigt verschiedene Ausgestaltungsformen, insbesondere Aussparungen in der ersten und oder zweiten Elektrodenschicht einer Vorrichtung zum Behandeln von Oberflächen, wie sie beispielsweise in den Fig. 1 und 2 dargestellt und beschrieben ist. Dargestellt sind verschiedene Ausgestaltungsformen mit jeweils einer ersten Elektrode 12, einer zweiten Elektrode 14 und einer Dielektrikumsschicht 13 zwischen der ersten und zweiten Elektrode 12, 14. Hierbei sind verschiedene Formen für Aussparungen 90 in der zweiten Elektrode14 gezeigt, beispielsweise lochförmige 91, streifenförmige 92, mäanderförmige 95, wabenförmige 94, kreisförmige 96 und/oder quadratische Aussparungen 93. Ohne Einschränkung der Erfindung kann es auch vorgesehen sein, dass beide, nämlich die erste und zweite Elektrode 12, 14, mit Aussparungen 90 in den verschiedensten Formen gebildet sind.

### Bezuqszeichenliste

- 1: Vorrichtung
- 2: Mehrschichtsystem
- 3: Zugewandte Seite der Vorrichtung 1
- 4: Abgewandte Seite der Vorrichtung 1
- 5: Kabel
- 11: Erste Isolierschicht
- 12: Erste Elektrodenschicht, insbesondere Hochspannungselektrodenschicht
- 13: Dielektrikumsschicht
- 14: Zweite Elektrodenschicht, insbesondere Masse-Elektrodenschicht
- 15: Zweite Isolierschicht
- 16: Abstandshalterschicht
- 17: Dritte Isolierschicht
- 30: Stecker
- 31: Unteres Steckergehäuse
- 32: Oberes Steckergehäuse
- 33: Klemmvorrichtung
- 34: Anschluss für die zweite Elektrodenschicht 14
- 35: Weiterer Anschluss
- 36: Anschluss für die erste Elektrodenschicht 12
- 37: Erste Klemmzunge
- 38: Zweite Klemmzunge
- 39: Hochspannungsanschluss
- 40: Masseanschluss
- 41: Kabelanschluss
- 42: Gelenk
- 70: Generatoreinheit
- 71: Display
- 80: Informationsträger
- 90: Aussparung in der ersten und/oder zweiten Elektrodenschicht
- 91: Lochförmige Aussparung
- 92: Streifenförmige Aussparung
- 93: Quadratische Aussparung
- 94: Wabenförmige Aussparung
- 95: Mäanderförmige Aussparung
- 96: Kreis- und/oder halbkreisförmige Aussparung
- 100: System
- A: Offene Position der Klemmvorrichtung 33
- B: Geschlossene Position der Klemmvorrichtung 33
- D2: Dicke des Mehrschichtsystems 2
- L2: Länge des Mehrschichtsystems 2
- B2: Breite des Mehrschichtsystems 2

## Patentansprüche

1. Vorrichtung (1) zum Erzeugen eines kalten Atmosphärendruckplasmas für die Behandlung von menschlichen und/oder tierischen Oberflächen, umfassend ein flexibles, flächiges Mehrschichtsystem (2) mit einer der zu behandelnden Oberfläche zugewandten Seite (3) und einer der zu behandelnden Oberfläche abgewandten Seite (4), wobei das Mehrschichtsystem (2) die folgenden Schichten umfasst:
- eine erste Elektrodenschicht (12) auf der abgewandten Seite (4) des Mehrschichtsystems (2),
- eine zweite Elektrodenschicht (14) auf der zugewandten Seite (3) des Mehrschichtsystems (2), wobei die zweite Elektrodenschicht (14) eine Vielzahl von Aussparungen (90) aufweist oder gitterartig oder mäanderförmig ausgebildet ist,
- eine Dielektrikumsschicht (13), die zwischen der ersten Elektrodenschicht (12) und der zweiten Elektrodenschicht (14) angeordnet ist,
- eine erste Isolierschicht (11), wobei die erste Isolierschicht (11) benachbart zur ersten Elektrodenschicht (12) auf der von der zu behandelnden Oberfläche abgewandten Seite (4) des Mehrschichtsystems (2) angeordnet ist,
- eine Abstandshalterschicht (16), wobei die Abstandshalterschicht (16) konfiguriert ist, ein ausreichendes Gasvolumen zum Zünden des Plasmas bereitzustellen, **dadurch gekennzeichnet, dass** das Mehrschichtsystem (2) zusätzlich eine zweite Isolierschicht (15) umfasst, wobei die zweite Isolierschicht (15) benachbart zu der zweiten Elektrodenschicht (14) auf der von der zu behandelnden Oberfläche zugewandten Seite (3) des Mehrschichtsystems (2) angeordnet ist und dass die Abstandshalterschicht (16) oberhalb auf der zweiten Isolierschicht (15) auf der zugewandten Seite (4) des Mehrschichtsystems (2) angeordnet ist.

2. Vorrichtung (1) gemäß Anspruch 1, **dadurch gekennzeichnet, dass** die zweite Isolierschicht (15) nicht integral mit der Abstandshalterschicht (16) gebildet ist.

3. Vorrichtung (1) nach einem der vorhergehenden Ansprüche, **dadurch gekennzeichnet, dass** die zweite Isolierschicht (15) eine Dicke zwischen 10 µm bis 300 µm aufweist.

4. Vorrichtung (1) nach einem der vorhergehenden Ansprüche, **dadurch gekennzeichnet, dass** das Mehrschichtsystem (2) zusätzlich eine dritte Isolierschicht (17) aufweist, wobei die dritte Isolierschicht (17) benachbart zu der Abstandshalterschicht (16) auf der von der zu behandelnden Oberfläche zugewandten Seite (3) des Mehrschichtsystems (2) angeordnet ist.

5. Vorrichtung (1) nach Anspruch 4, **dadurch gekennzeichnet, dass** die dritte Isolierschicht (17) eine Dicke zwischen 50 µm bis 300 µm, bevorzugt von 200 µm aufweist.

6. Vorrichtung (1) nach zumindest einem der vorhergehenden Ansprüche, **dadurch gekennzeichnet, dass** die erste Elektrodenschicht (12) durchgängig oder mit einer Vielzahl von Aussparungen gebildet ist.

7. Vorrichtung (1) nach zumindest einem der vorhergehenden Ansprüche, **dadurch gekennzeichnet, dass** die Aussparungen (90) in der zweiten Elektrodenschicht (14) loch- (91), streifen- (92), mäander- (95), waben- (94), kreisförmig (96) und/oder quadratisch (93) ausgebildet sind.

8. Vorrichtung (1) gemäß Anspruch 6, **dadurch gekennzeichnet, dass** die Aussparungen in der ersten Elektrodenschicht (12) loch- (91), streifen- (92), mäander- (95), waben-(94), kreisförmig und/oder quadratisch ausgebildet sind.

9. Vorrichtung (1) nach zumindest einem der vorhergehenden Ansprüche, **dadurch gekennzeichnet, dass** die Vorrichtung (1) einen Informationsträger (80), insbesondere einen Chip, ein Etikett und/oder ein sonstiges Informations- und Speichermedium, umfasst auf dem zumindest ein Betriebsparameter zum Betreiben der Vorrichtung (1) gespeichert ist.

10. System (100) mit einer Vorrichtung (1) nach zumindest einem der Ansprüche 1 bis 9, einem Kabel (5) zum Verbinden der Vorrichtung (1), wobei das Kabel (5) einen Stecker (30) aufweist, der konfiguriert ist, eine steckbare Hochspannungsverbindung zwischen der Vorrichtung (1) und dem Kabel (5) bereitzustellen, und einer Generatoreinheit (70) zum Bereitstellen einer Hochspannung zum Erzeugen eines kalten Atmosphärendruckplasmas mit der Vorrichtung (1), wobei die Generatoreinheit (70) konfiguriert ist, die Vorrichtung (1) zu steuern und Betriebsparameter zum Steuern der Vorrichtung (1) aus einem Informationsträger (80), insbesondere einem Chip, einem Etikett und/oder einem sonstigen Informations- und Speichermedium, in oder an der Vorrichtung (1) auszulesen.

11. System nach Anspruch 10, **dadurch gekennzeichnet, dass** das Kabel (5) eine Klemmvorrichtung (33) aufweist, und die Klemmvorrichtung (33) zwischen einer offenen Position (A) und einer geschlossenen Position (B) verlagerbar ist, wobei in der geschlossen Position (B) die Vorrichtung (1) elektrisch mit dem Kabel (5) verbunden und in der offenen Position (A) die Vorrichtung von dem Kabel (5) elektrisch getrennt ist.

## Claims

1. A device (1) for producing a cold atmospheric pressure plasma for the treatment of human and / or animal surfaces, comprising a flexible, planar multilayer system (2) with a side (3) facing the surface to be treated and a side (4) facing away from the surface to be treated, the multilayer system (2) comprising the following layers:
- a first electrode layer (12) on the side (4) facing away from the multilayer system (2),
- a second electrode layer (14) at the facing side (3) of the multilayer system (2), wherein the second electrode layer (14) has a plurality of recesses (90) or is formed in a grid-like or meander-like manner,
- a dielectric layer (13) arranged between the first electrode layer (12) and the second electrode layer (14),
- a first insulating layer (11), wherein the first insulating layer (11) is arranged adjacent to the first electrode layer (12) on the side (4) of the multilayer system (2) facing away from the surface to be treated,
- a spacer layer (16), wherein the spacer layer (16) is configured to provide a sufficient gas volume to ignite the plasma,
**characterized in that**
the multilayer system (2) additionally comprises a second insulating layer (15), wherein the second insulating layer (15) is arranged adjacent to the second electrode layer (14) on the side (3) of the multilayer system (2) facing the surface to be treated
and that
the spacer layer (16) is arranged above on the second insulating layer (15) on the facing side (4) of the multilayer system (2).

2. Device (1) according to claim 1, **characterized in that** the second insulating layer (15) is not integrally formed with the spacer layer (16).

3. Device (1) according to any of the preceding claims, **characterized in that** the second insulating layer (15) has a thickness between 10 µm to 300 µm.

4. Device (1) according to any of the preceding claims, **characterized in that** the multilayer system (2) additionally has a third insulating layer (17), wherein the third insulating layer (17) is arranged adjacent to the spacer layer (16) on the side (3) of the multilayer system (2) facing the surface to be treated.

5. Device (1) according to claim 4, **characterized in that** the third insulating layer (17) has a thickness between 50 µm to 300 µm, preferably of 200 µm.

6. Device (1) according to at least one of the preceding claims, **characterized in that** the first electrode layer (12) is formed continuously or with a plurality of recesses.

7. Device (1) according to at least one of the preceding claims, **characterized in that** the recesses (90) in the second electrode layer (14) are hole-shaped (91), strip-shaped (92), meander-shaped (95), honeycomb-shaped (94), circular-shaped (96) and/or square-shaped (93).

8. Device (1) according to claim 6, **characterized in that** the recesses in the first electrode layer (12) are hole-shaped (91), strip-shaped (92), meander-shaped (95), honeycomb-shaped (94), circular and/or square-shaped.

9. Device (1) according to at least one of the preceding claims, **characterized in that** the device (1) comprises an information carrier (80), in particular a chip, a label and/or another information and storage medium, on which at least one operating parameter for operating the device (1) is stored.

10. System (100) with a device (1) according to at least one of claims 1 to 9, a cable (5) for connecting the device (1), wherein the cable (5) has a plug (30) configured to provide a plug-in high voltage connection between the device (1) and the cable (5), and a generator unit (70) for providing a high voltage for generating a cold atmospheric pressure plasma with the device (1), wherein the generator unit (70) is configured to control the device (1) and to read out operating parameters for controlling the device (1) from an information carrier (80), in particular a chip, a label and/or another information and storage medium, in or on the device (1).

11. System according to claim 10, **characterized in that** the cable (5) has a clamping device (33), and the clamping device (33) can be displaced between an open position (A) and a closed position (B), wherein in the closed position (B) the device (1) is electrically connected to the cable (5) and in the open position (A) the device is electrically disconnected from the cable (5).

## Revendications

1. Dispositif (1) de génération d'un plasma froid à pression atmosphérique pour le traitement de surfaces humaines et/ou animales, comprenant un système multicouche plan flexible (2) avec un côté (3) tourné vers la surface à traiter et un côté (4) détourné de la surface à traiter, dans lequel le système multicouche (2) comprend les couches suivantes :
- une première couche d'électrode (12) sur le côté détourné (4) du système multicouche (2),
- une seconde couche d'électrode (14) sur le côté tourné (3) du système multicouche (2), dans lequel la seconde couche d'électrode (14) présente une pluralité d'évidements (90) ou est réalisée à la manière d'une grille ou en forme de méandre,
- une couche diélectrique (13) qui est disposée entre la première couche d'électrode (12) et la seconde couche d'électrode (14),
- une première couche isolante (11), dans lequel la première couche isolante (11) est disposée de manière voisine à la première couche d'électrode (12) sur le côté (4) du système multicouche (2) détourné de la surface à traiter,
- une couche d'écarteur (16), dans lequel la couche d'écarteur (16) est configurée pour mettre à disposition un volume gazeux suffisant pour l'allumage du plasma,
**caractérisé en ce que**
le système multicouche (2) comprend en outre une deuxième couche isolante (15), dans lequel la deuxième couche isolante (15) est disposée de manière voisine à la seconde couche d'électrode (14) sur le côté (3) du système multicouche (2) tourné vers la surface à traiter,
et que
la couche d'écarteur (16) est disposée sur le dessus de la deuxième couche isolante (15) sur le côté détourné (4) du système multicouche (2).

2. Dispositif (1) selon la revendication 1, **caractérisé en ce que** la deuxième couche isolante (15) n'est pas intégralement formée de la couche d'écarteur (16).

3. Dispositif (1) selon une des revendications précédentes, **caractérisé en ce que** la deuxième couche isolante (15) présente une épaisseur comprise entre 10 µm et 300 µm.

4. Dispositif (1) selon une des revendications précédentes, **caractérisé en ce que** le système multicouche (2) présente en outre une troisième couche isolante (17), dans lequel la troisième couche isolante (17) est disposée de manière voisine à la couche d'écarteur (16) sur le côté (3) du système multicouche (2) tourné vers la surface à traiter.

5. Dispositif (1) selon la revendication 4, **caractérisé en ce que** la troisième couche isolante (17) présente une épaisseur comprise entre 50 µm et 300 µm, de préférence de 200 µm.

6. Dispositif (1) selon au moins une des revendications précédentes, **caractérisé en ce que** la première couche d'électrode (12) est formée en continu ou d'une pluralité d'évidements.

7. Dispositif (1) selon au moins une des revendications précédentes, **caractérisé en ce que** les évidements (90) dans la seconde couche d'électrode (14) sont réalisés en forme de trou (91), bande (92), méandre (95), nid d'abeilles (94), de manière circulaire (96) et/ou carrée (93).

8. Dispositif (1) selon la revendication 6, **caractérisé en ce que** les évidements dans la première couche d'électrode (12) sont réalisés en forme de trou (91), bande (92), méandre (95), nid d'abeilles (94), de manière circulaire et/ou carrée.

9. Dispositif (1) selon au moins une des revendications précédentes, **caractérisé en ce que** le dispositif (1) comprend un support d'informations (80), notamment une puce, une étiquette et/ou un autre support d'informations et de stockage, sur lequel au moins un paramètre de service est mémorisé pour l'exploitation du dispositif (1).

10. Système (100) avec un dispositif (1) selon au moins une des revendications 1 à 9, un câble (5) pour la connexion du dispositif (1), dans lequel le câble (5) présente un connecteur (30) qui est configuré pour mettre à disposition une connexion enfichable haute tension entre le dispositif (1) et le câble (5), et un module de générateur (70) pour la mise à disposition d'une haute tension en vue de la génération d'un plasma froid à pression atmosphérique avec le dispositif (1), dans lequel le module de générateur (70) est configuré pour commander le dispositif (1) et lire des paramètres de service pour la commande du dispositif (1) à partir d'un support d'informations (80), notamment d'une puce, d'une étiquette et/ou d'un autre support d'informations et de stockage, dans ou sur le dispositif (1).

11. Système selon la revendication 10, **caractérisé en ce que** le câble (5) présente un dispositif de serrage (33), et le dispositif de serrage (33) peut être déplacé entre une position ouverte (A) et une position fermée (B), dans lequel le dispositif (1) est connecté électriquement au câble (5) dans la position fermée (B) et le dispositif est séparé électriquement du câble (5) dans la position ouverte (A).
